(19) Europäisches Patentamt / European Patent Office / Office européen des brevets

(11) **EP 4 132 347 B1**

(12) **EUROPEAN PATENT SPECIFICATION**

(45) Date of publication and mention
of the grant of the patent:
**01.05.2024 Bulletin 2024/18**

(51) International Patent Classification (IPC):
**A61B 5/00** (2006.01)     **G01N 21/64** (2006.01)
**G01N 33/573** (2006.01)

(21) Application number: **21720180.5**

(52) Cooperative Patent Classification (CPC):
**A61B 5/0071; A61B 5/0062; A61B 5/0073;
G01N 33/5735; G01N 33/57484**

(22) Date of filing: **07.04.2021**

(86) International application number:
**PCT/EP2021/059034**

(87) International publication number:
**WO 2021/204858 (14.10.2021 Gazette 2021/41)**

(54) **METHOD FOR QUANTITATIVE IMAGING OF THE METABOLIC STATUS OF A LESION**

VERFAHREN ZUR QUANTITATIVEN DARSTELLUNG DES STOFFWECHSELSTATUS EINER LÄSION

PROCÉDÉ D'IMAGERIE QUANTITATIVE DE L'ÉTAT MÉTABOLIQUE D'UNE LÉSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.04.2020 EP 20169219**

(43) Date of publication of application:
**15.02.2023 Bulletin 2023/07**

(73) Proprietor: **Humanitas Mirasole S.p.A.
20089 Rozzano (MI) (IT)**

(72) Inventors:
• **DONI, Andrea**
  **20089 ROZZANO (MI) (IT)**
• **MORONE, Diego**
  **20089 ROZZANO (MI) (IT)**

(74) Representative: **Ghirardi, Valeria et al
De Simone & Partners S.r.l.
Via Vincenzo Bellini, 20
00198 Roma (IT)**

(56) References cited:
**US-A1- 2012 276 578**

• **RANAWAT HARSH ET AL: "Recent trends in two-photon auto-fluorescence lifetime imaging (2P-FLIM) and its biomedical applications", BIOMEDICAL ENGINEERING LETTERS, THE KOREAN SOCIETY OF MEDICAL AND BIOLOGICAL ENGINEERING, KOREA, vol. 9, no. 3, 1 July 2019 (2019-07-01), pages 293-310, XP036863134, ISSN: 2093-9868, DOI: 10.1007/S13534-019-00119-7 [retrieved on 2019-07-01]**
• **HANSON KERRY M ET AL: "Two-photon fluorescence lifetime imaging of the skin stratum corneum pH gradient", BIOPHYSICAL JOURNAL, ELSEVIER, AMSTERDAM, NL, vol. 83, no. 3, 1 September 2002 (2002-09-01), pages 1682-1690, XP002636887, ISSN: 0006-3495, DOI: 10.1016/S0006-3495(02)73936-2**
• **LARS ALEXANDER SCHNEIDER ET AL: "Influence of pH on woundhealing: a new perspective for wound-therapy?", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 298, no. 9, 8 November 2006 (2006-11-08), pages 413-420, XP019473786, ISSN: 1432-069X**

## Description

This invention pertains to a fluorescence lifetime imaging microscopy (FLIM) -based method for the quantitative three-dimensional (3D) imaging of the metabolic status of a lesion, comprising the acquisition of FLIM data of a least one anaerobic glycolysis marker. This method applies in particular to lesions such as wounds and tumours, and to markers such as the NADH/NAD+ ratio, the NAD(P)H/NAD+ ratio, the NADH/FAD ratio, the NAD(P)H/FAD ratio or the FADH/FAD ratio as a first marker, and their optional combination with a second marker such as pH, oxygen tension, glucose, pyruvate and lactate.

## BACKGROUND

In normal skin, dermis has an extracellular neutral-alkaline pH[1,2]. After tissue damage, cells involved in tissue repair undergo a metabolic adaptation resulting from low oxygen tension towards a less energy-efficient process of anaerobic metabolism that leads to microenvironment acidosis[2,3]. In *vitro* evidence suggests a beneficial effect of acidic pH in several processes implicated in wound healing, which involve cell adhesion[4,5], migration and proliferation[6]. In a previous report[7], we described an unprecedented role of tissue acidification in setting PTX3, a key component of the humoral arm of innate immunity, in a tissue remodelling and repair mode. In wounds, reduced oxygen tension[8] and factors downstream of cell anaerobic glycolysis, such as lactate[9,10], effectively stimulate immune and vascular endothelial cells to release factors that support angiogenesis. Subsequent neovascularization allows restoration of nutrient delivery and oxygen, and cells use oxidative metabolism for their longer-term functions contributing to restore the wound pH to values near to neutral[2,3].

While an acidic pH occurs in the inflammatory phases of acute wounds that progress on healing, chronic and highly infected wounds are characterized by abundant recruitment of neutrophils and a non-acidic pH[11,12]. Chronic non-healing wounds may occur secondarily to a high alkaline pH[11,13], and studies report a relationship between wound pH and chronic wound healing[14,15,16]. The effect of acidic pH in the wound bed has a clinical functional relevance on the healing of chronic wounds[11]. In fact, a prolonged chemical acidification of the wound bed increases the healing rate in chronic venous leg ulcers[17]. Chronic non-healing wounds continue to represent a therapeutic challenge for clinicians. Indeed, measurement of wound pH predicts healing outcomes and skin graft survival in experimental and clinical studies and determines success or failure of surgical treatment of burns and chronic ulcers[11, 17]. Measurement of skin pH is usually carried out with pH electrodes, which is limited to the skin surface or in the evaluation of superficial environmental factors[13] and provides an inherently low spatial resolution. The measurement in the inner parts of the skin can be attempted by the subsequent removal of skin layers with adhesive tape[18], however applied with limitation to measurement in the damaged dermis.

In tumours, a high metabolic rate leads to acidosis in poorly perfused regions as a result of high glucose consumption and high lactate production[9,19,20], thus affecting cancer progression[10,21,22,23]. Acidic tumour microenvironment induces cancer cells to increase formation of lamellipodia, adhesion and invasiveness, as well as the increased secretion of ECM proteases[24,25,26,27]. A possible molecular mechanism underlying the promotion of cell adhesion and invasion was related to the pH-dependent activation of cell surface integrins[5]. Interference with pH may represent a therapeutic target also in cancer[28]. Macrophages have a direct impact on metabolism in the tumour microenvironment[10,29]. M1-polarized macrophages show a metabolic shift towards an anaerobic glycolytic pathway typical in acute infections and hypoxic tissues, and iron storage through ferritin expression. On the other hand, M2-polarized macrophages show an oxidative glucose metabolism and express high levels of ferroportin, an iron exporter that promotes tissue repair and tumour growth[29,30]. The definition of mechanisms that regulate the metabolic activity of macrophages may be important to define their relevance in cancer progression[10].

US 2012/276578 A1 discloses a method for discriminating the in vivo metabolic state of cells in a tissue comprising providing a tissue sample comprising a plurality of tissue components; performing fluorescence lifetime imaging microscopy to said tissue sample to generate a fluorescence lifetime imaging data of said tissue; and performing image segmentation to measure the average phasor value of regions of interest in the tissues, whereby the relative concentration of the tissue components are determined. Tissues or tissue samples utilized can be cancer or precancer cells. Tissue components may be NADH/NAD+ ratio.

## BRIEF DESCRIPTION OF THE FIGURES

**Figure 1: Kinetic analysis (left) and representative macroscopic images (right) of untreated vs DCA-treated skin wounds**

**Figure 2: Measurement of anaerobic glycolysis in wound by FLIM quantification of NADH/NAD+ ratio.** (A)map of a gradient of NADH/NAD+ ratio .(B) phasor analysis of FLIM NADH fluorescence.. (C) representative portion of a straightened image of NADH/NAD+ ratio from wound margin (D), NADH/NAD+ ratio percentage as a function of distance from wound (E), *XZ* maximum intensity projection around the wound margin.

**Figure 3: Analysis of hypoxia in wound.** (A) Pimonidazole fluorescence intensity image of a wound. (B), Pimonidazole fluorescence intensity image straightened along wound margin (C), XZ maximum intensity projection of Pimonidazole fluorescence intensity. (D), Pimonidazole intensity as a function of distance from the perilesional area.

**Figure 4: Analysis of pH in wound**. (A) maximum intensity projection of a representative pH map stack.. (B) linearized portion of a slice of the same pH map stack. (C) XZ maximum intensity projection. (D), pH as a function of distance from the wound.

**Figure 5: Measurement of anaerobic glycolysis in the tumor microenvironment by FLIM quantification of NADH/NAD⁺ ratio** (A) (A) 740 nm Photomultiplier tube (PMT) imaging (B), NADH/NAD⁺ ratio map. (C), representative portion of a straightened NADH/NAD⁺ ratio map. (D)NADH/NAD⁺ ratio as a function of distance from the tumor front.

**Figure 6. Views of skinfold chamber.**

**Figure 7. Two-phase exponential decay fit of NADH lifetime profile of untreated vs DCA treated skin wounds.**

**Figure 8 Effect of the mitochondrial respiratory chain in the measurement of NADH/NAD⁺ ratio as expression of glycolysis in wound**. (A) NADH/NAD⁺ ratio percentage as a function of distance from wound. (B) map of a gradient of NADH/NAD+ ratio in wound in untreated or mice treated with Rotenone and Antimycin A.

## DESCRIPTION OF THE INVENTION

There is provided a method for the quantitative three-dimensional (3D) imaging of the metabolic status of a lesion, such method comprising:

a) acquiring fluorescence lifetime imaging microscopy (FLIM) data of an anaerobic glycolysis marker of the lesion

b) subjecting the data relative to the anaerobic glycolysis marker acquired in step a) to a phasor analysis, thereby obtaining a phasor diagram

c) using the phasor diagram obtained on step b) to computationally generate a quantitative 3D image of the anaerobic glycolysis status of the lesion, wherein the anaerobic glycolysis marker is selected from the list of the NADH/NAD+ ratio, NAD(P)H/NAD+ ratio, the NADH/FAD ratio, the NAD(P)H/FAD ratio and the FADH/FAD ratio, and wherein step c) when applied to the data relative to anaerobic glycolysis marker consists in dividing the data in a number of regions corresponding to different intervals of NADH, NAD(P)H or FADH lifetimes and assigning each pixel belonging to the same region a single intensity value correlated to such lifetime interval.

In another embodiment, the method further comprises the following steps:

d) acquiring, simultaneously to the data acquired in step a), FLIM data relative to a second metabolic marker of the lesion;

e) computationally generate a quantitative 3D image with the data acquired in step d)

f) overlay the images obtained in steps c) and e) thereby obtaining a 3D image of the metabolic status of a lesion

In one embodiment, the second metabolic marker is selected from the list of pH, oxygen tension, glucose, pyruvate and lactate.

In another e embodiment, the number of regions is chosen from the list of at least 3, at least 5 and 5.

In another embodiment in step e) the data relative to pH is processed by fitting the fluorescence lifetime of each pixel to a pH value by reference to a standard calibration scale.

In yet another embodiment, the FLIM is selected from the list of single photon FLIM, 2-photon FLIM and multiphoton FLIM.

In a further embodiment, the lesion is selected from the list of a wound, an internal wound, a cutaneous wound, a pathologic wound, an ulcer, a scar, a solid tumour and a tumour-draining lymph node.

In another embodiment, the solid tumour is selected from the list of skin tumour, melanoma, colon tumour, soft tissue sarcoma, visceral sarcoma, fibrosarcoma, liver tumour and stomach tumour.

All embodiments may be combined.

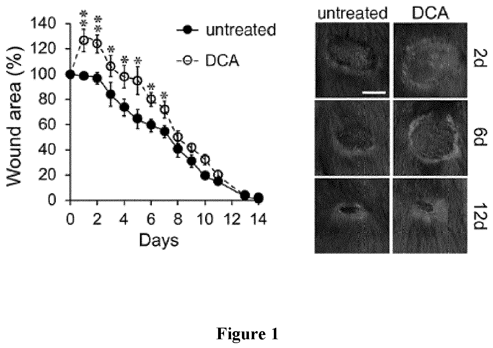

The invention may be further described by the results discussed below, in reference to figures 1 to 8 .

## RESULTS

**Figure 1:** *In vivo* **relevance of the cell metabolic adaptation in skin repair.** The excisional skin wound healing model was used. Left, kinetic analysis of skin wound areas was performed. Values represent mean $\pm$ SD. Right, representative macroscopic images of untreated and DCA treated mice are shown at the indicated days after wounding. Bar, 5 mm. *P < 0.05, **P < 0.01; Student's *t*-test. Untreated, n=8; DCA, n=9. One experiment shown out of two performed with similar number of animals.

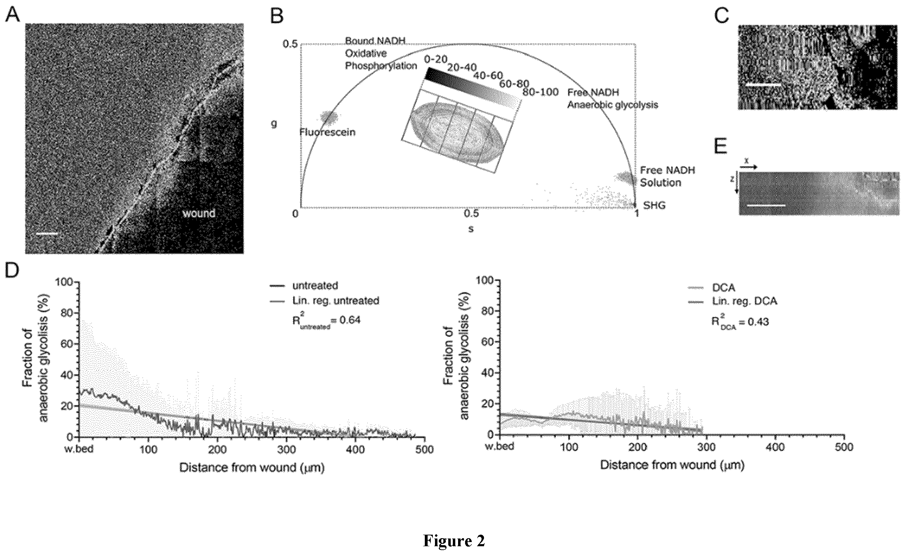

**Figure 2: Measurement of anaerobic glycolysis in wound by FLIM quantification of NADH/NAD⁺ ratio.** Phasor analysis of free and bound NADH fluorescence allows a quantification of their relative increase. A, map of a gradient of NADH/NAD+ ratio (grey). Wound margin is highlighted with a dashed line. All scale bars 100$\mu$m. B, phasor analysis of FLIM NADH fluorescence. NADH region of the phasor plot was divided in five subregions along the major axis and each is associated with an intensity value (0-20, 20-40, 40-60, 60-80, 80-100). Reference calibration points for SHG, to account for the IRF, and for fluorescein and NADH are also displayed (n=5 measurements for each performed). C, the image shows a representative portion of a straightened image of NADH/NAD$^+$ ratio from wound margin (dashed line). D, NADH/NAD$^+$ ratio percentage as a function of distance from wound (grey). The graph shows the increase of NADH production around the wound. Linear regression with 95% confidence level bands (black line) shows a significant decrease in the ratio from the wound front (Deviation from slope zero test, P<0.0001); n=11 mice. NADH/NAD$^+$ ratio is lower in perilesional area of DCA-treated mice (grey line vs grey line of D, comparison between first 10 values of each condition, $t$-test ****, P<0.0001). n=3 mice. Comparison between linear regressions with 95% confidence level bands (darker lines with bands) show a reduction in line slope in DCA-treated mice (Deviation between slopes test, P<0.0001. Deviation from slope zero test, P<0.0001). E, $XZ$ maximum intensity projection shows a preferential increase of anaerobic glycolysis (higher intensity) around the wound margin.

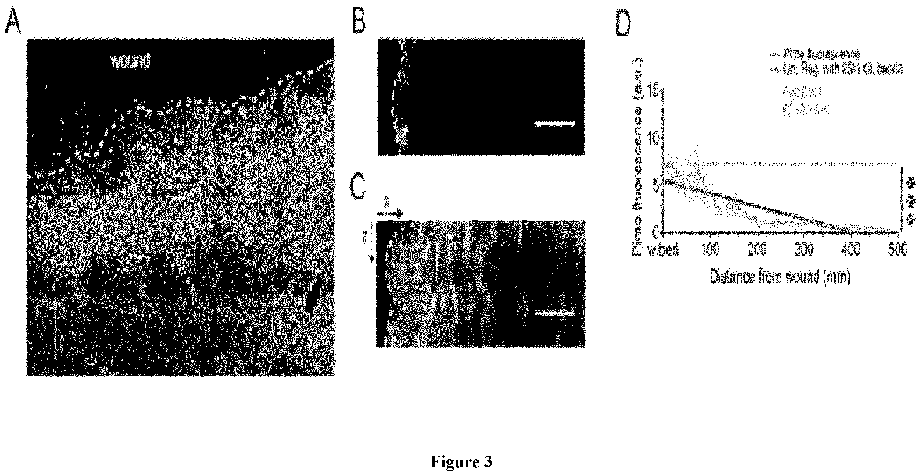

**Figure 3: Analysis of hypoxia in wound.** A, the image shows a preferential localization of hypoxia (larger white spots around the wound edge) in regions of higher glycolytic metabolism (increased NADH/NAD$^+$ ratio; grey). Pimonidazole (Pimo) fluorescence intensity was used to detect hypoxic regions. Wound margin is highlighted with a dashed line. All scale bars 100$\mu$m. B, Pimo image straightened along wound margin (dashed line). C, $XZ$ maximum intensity projection of Pimo fluorescence intensity. D, Pimo intensity as a function of distance from the perilesional area. Linear regression 95% confidence level bands show a significant decrease (Deviation from slope zero test, P<0.0001). n=3 mice.

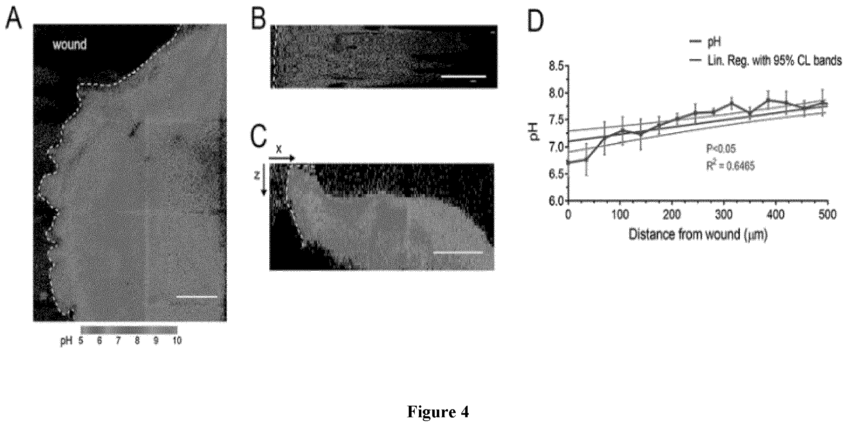

**Figure 4: Analysis of pH in wound**. A, maximum intensity projection of a representative pH map stack. Gray gradient represents pH as indicated in the pH scale. Wound margin is highlighted with a dashed line. All scale bars 100 $\mu$m. B, linearized portion of a slice of the same pH map stack. C, $XZ$ maximum intensity projection. D, pH as a function of distance from the wound. Images from B were averaged. Linear regression with 95% confidence level bands shows a significant increase (Deviation from slope zero test, P<0.05). n=6 mice.

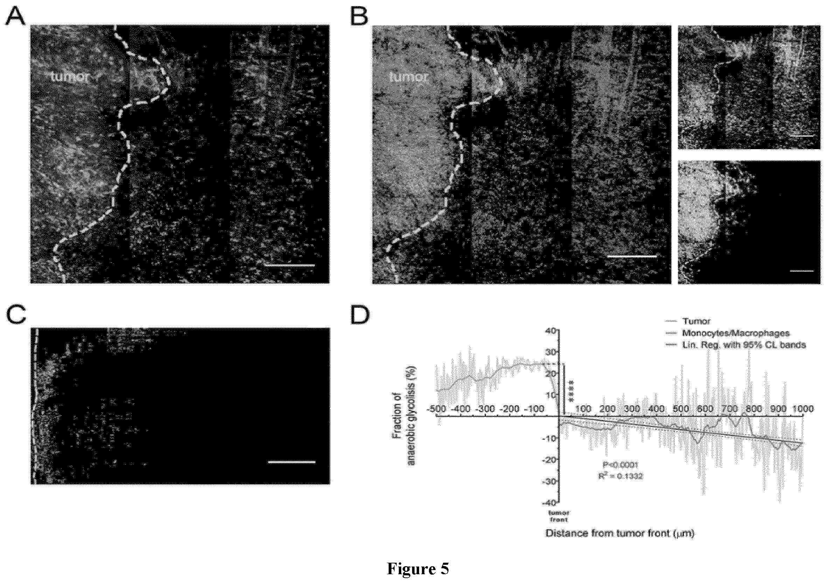

**Figure 5: Measurement of anaerobic glycolysis in the tumor microenvironment by FLIM quantification of NADH/NAD$^+$ ratio.** CX3CR1$^{+/gfp}$ mouse monocytes were i.d. injected 1-day prior acquisition. A, stack acquired with PMTs is used to prepare a binary mask of the two cell populations. Dashed line highlights tumor front. All scale bars 200$\mu$m. B, binary mask is applied to the NADH/NAD$^+$ map to obtain a map subset for each cell population. C, representative portion of a straightened NADH/NAD$^+$ ratio map for the GFP$^+$ cells. D, NADH/NAD$^+$ ratio as a function of distance from the tumor front. Solid brighter lines were obtained by application of a LOWESS smoothing. Linear regression of the GFP$^+$ cells (darkest line with 95% confidence level bands) shows a decrease in NADH/NAD$^+$ ratio in CX3CR1$^{+/gfp}$ cells from the tumor front (deviation from slope zero test, P<0.0001), while a Student $t$-test between the two populations shows that tumor cells globally have higher NADH/NAD$^+$ ratio compared to the CX3CR1$^{+/gfp}$ cells. **** P< 0.0001. n=6 independent mice.

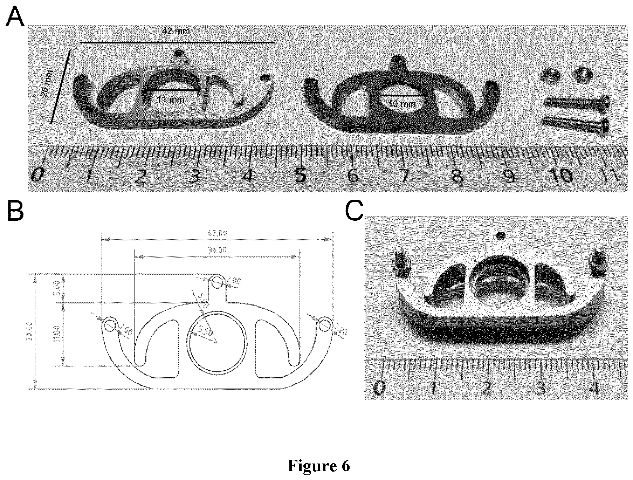

**Figure 6. View of skinfold chamber.** A, the chamber shown from the exterior (left) and interior (right) part, with screws and bolts for mounting. The chamber has an imaging window of 10 mm. To further preserve the sample, a coverslip of size 11 mm can be mounted and fixed with an O-ring of same size. B, schematics of the chamber with dimensions. C, mounted chamber.

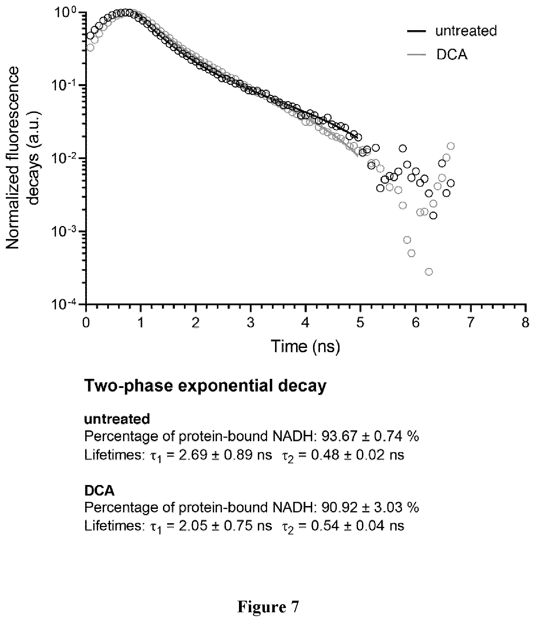

**Figure 7. Two-phase exponential decay fit of NADH lifetime profile.** Lifetime fit with a two-exponential decay curve of a perilesional region from single plane acquired with the same setting as Fig. 2 displays a short and long lifetime component, corresponding to free and proton-bound NADH, but fails to detect differences in the contributions. This approach is time consuming and can be misleading because free and protein-bound NADH have common exponential components and the NADH binding sites with different enzymes can not considered.

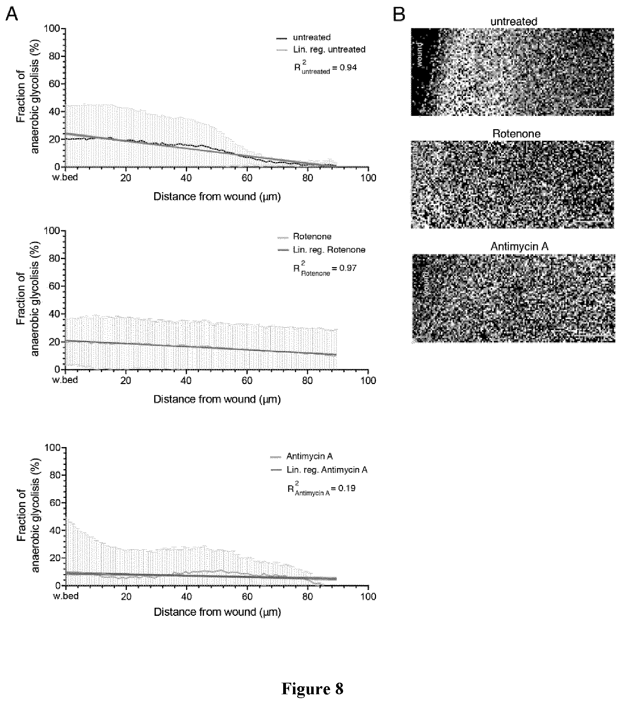

**Figure 8. Effect of the mitochondrial respiratory chain in the measurement of NADH/NAD$^+$ ratio as expression of glycolysis in wound.** A, NADH/NAD$^+$ ratio percentage as a function of distance from wound. Upon administration of Rotenone (middle graph vs grey graph for untreated condition), NADH/NAD$^+$ ratio shows no significant decrease in value (comparison between first 10 values of each condition, $t$-test, P>0.05), but a significant reduction in line slope (Deviation between slopes test, P<0.0001. Deviation from zero slope test, P<0.0001). NADH/NAD$^+$ ratio is lower in perilesional area of Antimycin A-treated mice (bottom graph vs grey line for untreated condition, comparison between first 10 values of each condition, $t$-test, P<0.0001). Comparison with linear regressions with 95% confidence level bands (darker lines with bands) show a reduction in line slope in Antimycin A-treated mice (Deviation between slopes test, P<0.0001. Deviation from slope zero test, P<0.0001). B, map of a gradient of NADH/NAD+ ratio (grey) in wound in untreated (n=4) or mice treated with Rotenone (n=3) and Antimycin A (n=2). Wound margin is highlighted with a dashed line. All scale bars 20$\mu$m.

**Relevance of cell metabolic adaptation in wound healing *in vivo***

Evidences suggest that wound acidification resulting from the cell metabolic adaptation after damage acts as a regulator of several activities involved in tissue repair[11, 14,16]. In order to determine the relevance of wound acidification in healing outcome *in vivo,* the pyruvate dehydrogenase kinase (Pdk) inhibitor dichloroacetate (DCA) was used in a model of full-thickness excisional skin wounding to promote glucose oxidation over anaerobic glycolysis, thus interfering with local acidosis.

As shown in Fig. 1, mice treated (n=9) with a single administration of DCA (300mg/Kg) 1h prior skin wounding showed significantly delayed curve compared to mice treated with vehicle alone (n=8). As assessed by measurement of wound area, DCA-mediated delayed wound closure corresponded to: day 1, -28.3% (P=0.005); day 2, -27.1% (P=0.004); day 3, -22.5% (P=0.05); day 4, -24.5% (P=0.02); day 5, -30.5% (P=0.002); day 6, -20.4% (P=0.01); day 7, -17.4% (P=0.03); compared to control mice (Fig. 1), thus inhibition of cellular anaerobic glycolysis at early stages significantly affects the process of tissue repair.

## Measurement of cell glycolysis activity in wound by FLIM

Relevance of the results *in vivo* prompted us to draft a method for investigating the cellular metabolic adaptation that occurs in tissue repair. Anaerobic glycolysis leads to an imbalance of the metabolic coenzyme NADH, as main electron exchanger in the glucose cycle, over $NAD^+$. However, NADH loses fluorescence intensity upon oxidation to $NAD^+$. Furthermore, fluorescence intensity measurements are subject to variations in concentration and quantum yield differences between free and protein-bound forms of NADH. Therefore, the fluorescence lifetime decay of the metabolic coenzyme NADH was measured to evaluate the cell glycolysis activity in wounded skin *in vivo.* An experimental model of dorsal full-thickness incisional wounding[7] and a custom-designed imaging chamber (Fig. 6) were used for the purpose.

On day 1 after wounding, FLIM images were acquired with 2PM and stitched to produce lifetime *XYZ* mosaics (600-1000$\mu$m wide and 200$\mu$m deep) (Fig. 2a). Common FLIM imaging has slow acquisition times, which are required to collect enough photons to reconstruct a time decay curve. This was compensated by the use of a time correlated single-photon counting (TCSPC) 16-channel parallel detector, with an average counting speed of 80 MHz which allowed faster 3D scanning of the mosaic over lesion area[31]. The ratio of free to protein-bound NADH has been known to be associated with the ratio of $NAD^+$ to NADH in small intestine epithelial cells[32] and breast cancer cells[33]. Protein-bound NADH has a complex multi-exponential lifetime decay that has been related to its binding to different enzymes, such as malate dehydrogenase and lactate dehydrogenase[34]. Standard approaches revolved on a characterization by fitting with a two-exponential curve, corresponding to a short or long lifetime range. This approximation is however misleading because free and protein-bound NADH have common exponential components and the NADH binding sites with different enzymes cannot be considered[32,35]. In fact, in our case this approach was ineffective to discriminate between untreated and DCA-treated mice (Fig. 7). Lifetime decays in perilesional regions of untreated and DCA-treated mice could not show significant differences when fitted with a two-exponential decay function (Fig. 7; Student t-test, P>0.05). Instead, phasor analysis[32,36] was used. In accordance with a previous report[37], we designed an analysis that first divides the phasor cloud into five regions (Fig. 2b) corresponding to a gradient of prevailing anaerobic glycolysis in regions of lower lifetimes towards a region where more oxidative phosphorylation occurs. Pixels attributed to each region were then associated to discrete values of decreasing intensity, and therefore quantification of pixel intensity on the resulting image was proportional to the relative increase in anaerobic glycolysis. By linearizing images, we obtained a spatial distribution of anaerobic glycolysis from wound profile (Fig. 2c). At lesion margin, gradient of NADH/$NAD^+$ ratio corresponded to 29.1$\pm$0.4% and decreased until to 2.6$\pm$0.1% distance (range of 200-500$\mu$m) from the wound. The decrease was evaluated with a linear regression fitting as the simplest fitting model for testing the overall deviation from zero slope curve (linear regression fitting with a slope of -0.050 $\pm$ 0.0016 $\mu$m$^{-1}$; black line with 95% CL bands; deviation from zero slope test, P<0.0001) (Fig. 2d). An increased anaerobic glycolysis around wound margins was also evident in *XZ* maximum intensity projection images of the maps (Fig. 2e). Administration of DCA (300 mg/Kg, i.p.; 1h) altered the glycolysis activity in wounds as assessed by phasor analysis of NADH (Fig. 2d, red). FLIM images were acquired in wounds of untreated mice and after injection of DCA. Reduction in anaerobic glycolysis at perilesional area resulted from 29.1$\pm$0.4 % to 8.0$\pm$0.2 % (mean of the first 10 values, *t*-test P<0.0001). Also, gradient steepness evaluated with linear regression fitting was lowered from a slope of -0.050 $\pm$ 0.0016 $\mu$m$^{-1}$ to -0.035 $\pm$ 0.0023 $\mu$m$^{-1}$ (Fig. 2d, darker lines with 95% CL bands. Deviation between slopes test, P<0.0001. Deviation from zero slope test for both conditions, P<0.0001). To assess the effect of the mitochondrial respiratory chain on glycolytic gradient in wound, Rotenone and Antimycin A were used *in vivo* as inhibitors of the complexes I and III of the Electron Transport Chain (ETC), respectively. Rotenone was reported to reduce NADH lifetimes and increase accumulation of free NADH[38]. Antimycin A binds to the Qi site of cytochrome c reductase affecting the terminal respiratory chain and hence ATP production. Local administration of Rotenone (20$\mu$M) impaired the formation of a gradient of NADH/$NAD^+$ ratio, lowering the slope from -0.267 $\pm$ 0.06 $\mu$m$^{-1}$ to -0.113 $\pm$ 0.002 $\mu$m$^{-1}$ (Fig. 8, darker lines with 95% CL bands. Deviation between slopes test, P<0.0001, n=3 per group). Rotenone did not affect the fraction of anaerobic glycolysis at wound margin (from 20.28 $\pm$ 0.41 % to 20.08 $\pm$ 0.21 %, Student t-test P>0.05) but it increased glycolysis in regions distant from the wound where an oxidative phosphorylation possibly occurs. Evidence are in line with previous reports[39,40], where Rotenone leads a metabolic shift towards NADH accumulation and glycolysis from oxidative phosphorylation. Antimycin A (5$\mu$M) altered the formation of a gradient (Fig. 8, slope decreased to -0.046 $\pm$ 0.08 $\mu$m$^{-1}$; de-

viation between slopes test, P<0.0001, untreated, n=3; Antimycin A, n=2) affecting, as expected[41], glycolysis at wound margin (8.37 ± 0.49 % (Student t-test, P<0.0001).

In the same wound model, the classical probes Hypoxyprobe Pimonidazole-HCl (Pimo) and 2', 7'-Bis-(2-Carboxyethyl)-5-(and-6)-Carboxyfluorescein (BCECF) were used to correlate glycolytic activity with regions of hypoxia and extracellular acidic pH, respectively, in wounds.

As assessed by measurement of Pimo fluorescence intensity, hypoxic areas were preferentially associated with increased NADH/NAD$^+$ ratio (Fig. 3a) at wound margins (Fig. 3b and 3c). Linear regression of Pimo fluorescence intensity fitted with a slope of -0.0134 ± 0.00032 $\mu$m$^{-1}$ (Fig. 3d, darker purple line with 95% CL bands. Deviation from zero slope test, P<0.0001).

The pH sensitive probe BCECF shows reduced emission intensity and lifetime in a protonated form. As reported[1], BCECF FLIM images were fitted with a single exponential after subtraction of an estimated instrument response function (IRF). These images were then stitched to produce lifetime XYZ mosaics (600-1000$\mu$m wide), from which we computed pH maps (Fig. 4a). To analyse the spatial distribution from the wound margin, the wound profile in the mosaics was then linearized (Fig. 4b) and pH was analysed as average in consecutive and equally distributed ROI bins from wound margin. XZ projections showed an acidic pH at the perilesional margins of 6.17±0.58, which at 200±35$\mu$m switches to a more basic pH of 7.82 ± 0.24 (Fig. 4c). The pH curve showed a significant increase in pH as a function of distance in $\mu$m from the wound margin, with a positive slope of 0.0013 ± 0.00022 $\mu$m$^{-1}$ when fitted with a linear regression model (Fig. 4d, darker green line with 95% CL bands. Deviation from zero slope test, P<0.05). Therefore, results indicate a correlation between regions of increased NADH/NAD$^+$ ratio as expression of augmented cell glycolytic activity ratio and hypoxia and acidification at wound margins.

**Measurement of cell glycolysis activity in cancer microenvironment by FLIM**

Solid tumours show an acidic microenvironment, which could be a determining factor in the induction of aggressive cancer phenotypes[20,26]. Metabolic adaptation in cancer is a key component of macrophage plasticity and definition of mechanisms regulating metabolic activity of macrophages, as well as the orchestration of metabolism by macrophages, are essential for the disease progression and represent a possible therapeutic intervention[10,23]. Measurement of NADH fluorescence lifetime was therefore extended in an experimental transplantable model of cancer in order to reconstruct a cellular metabolic map of macrophages in the tumour microenvironment (Fig. 5). mCherry-tagged cancer cell line CT26 were intradermally (i.d.) injected into CD-1 nude mice. On day 3, GFP$^+$ monocytes isolated from the spleen of CX3CR1$^{+/gfp}$ mice were i.d. injected in the peritumoral area. On day 4, dermis was surgically exposed with a skin flap and mounted into the imaging chamber with tumour at the centre. A first acquisition in standard imaging modality at 740 nm (Fig. 5a) was conducted to visualize the two cell populations and create their corresponding threshold-based binary masks. Thereafter, same XYZ area was then acquired in FLIM modality at 740 nm and analysed to obtain a NADH/NAD$^+$ map of the tumour and peritumoral area. The two binary masks were then used to extract NADH/NAD$^+$ maps of the corresponding cell populations (Fig. 5b). Images were then linearized from the tumour front to the peritumoral region for the GFP$^+$ population (Fig. 5c), and from the tumour front to the inner region of the lesion for the mCherry$^+$ population. As shown, at tumour-stromal interface mCherry$^+$ and GFP$^+$ cells showed a significantly higher NADH/NAD$^+$ ratio corresponding to a higher level of anaerobic glycolysis compared to the surrounding tissue (P<0.0001). At peritumoral region, linear regression (Fig. 5d, darker green line with 95% CL bands) of the NADH/NAD$^+$ ratio associated with GFP$^+$ cells showed a significant decrease (slope -0.0041 ± 0.00047 $\mu$m$^{-1}$, deviation from zero slope test P<0.0001).

**DISCUSSION**

We ascertained the effective relevance in vivo of cellular metabolic adaptation occurring in skin repair by interfering with anaerobic glycolytic activity. Moreover, we describe a protocol based on measurement of the intrinsic lifetime of NADH to reconstruct a spatial distribution of changes in anaerobic glycolysis correlated with areas of acidic pH and hypoxia in wound, as well as in a cancer. The method was challenged by interfering with the anaerobic glycolysis through the use of DCA, an inhibitor of Pdk that leads to enhanced activity of pyruvate dehydrogenase thereby augmenting oxidative phosphorylation[42]. Moreover, we report evidence of modulation of the glycolytic gradient of the wound by interfering with the mitochondrial respiratory chain through the use of a specific inhibitors.

In preclinical studies, the in vivo metabolic fluorescence imaging indirectly measures changes in oxygen tension, pH and glucose absorption in injured tissues and in tumour microenvironment by methods based on the detection of fluorescence intensity of specific sensitive probes, which suffer from local changes in probe concentrations and scattering. Ratiometric pH probes, such as seminaphtharhodafluor (SNARF), are intracellular pH indicators and are based on the intensity measurement at two emission wavelengths for the protonated and unprotonated forms of the dye[43]. Wavelengthdependent dyes can suffer from significant errors due to chromatic aberrations that can affect measurement especially in deeper portions of thick tissues[44]. Probes for optical in vivo imaging and positron-emission tomography (PET) exist to assess hypoxia[45] and glucose[46].

Therefore, combination between optical *in vivo* imaging and specific fluorescent probes is suitable for quantifying the selected markers over a period of time, being also minimally invasive. However, it lacks appropriate spatial resolution: on a readily accessible organ such as the skin, the commonly achievable resolution ranges from 5 to 1 mm. Measurement of intrinsic lifetime of NADH and FAD is emerging as a reliable technique for assessing cellular metabolism parameters *in vitro*[38]. NADH FLIM also enabled analysis of pharmacologically-induced alterations to mitochondrial metabolic processes from baseline cerebral metabolism in rat cerebral cortex[35] and metabolic changes at front of wound re-epithelialization in diabetic and control mice[47]. This prompted us to apply protocol for measuring the spatial distribution of NADH in experimental models of tissue repair, as well as of cancer. Indeed, fluorescence decay time is an intrinsic quantity and quantification of FLIM is not subject to the same limitations as measurement of fluorescence intensity[32]. Moreover, 2PM excitation provides high penetration depth into the tissues for intravital imaging and cell resolution, allowing functional three-dimensional imaging.

In the clinic, the general method for the evaluation of skin pH is limited to measurements with electrodes[13,18], which does not ensure adequate spatial resolution at the cellular level and a measurement limited to the most superficial part of the skin. Measurement of pH in non-healing or highly infected chronic wounds is an important element in the prediction of healing, as is the survival of skin graft in experimental and clinical studies and determines the success or failure of surgical treatment of burns and chronic ulcers[11,14,17]. Acidification of chronic venous ulcers is functionally relevant to the activities of remodelling cells, and therefore to their healing[11,16]. Overall, we describe a method that allows drawing a complete picture of wound metabolic phenotype and its spatial distribution, which could enhance the understanding of the relationship between microenvironment, metabolism and cell players in tissue repair. Moreover, this method could also represent a novel non-invasive diagnostic protocol for the monitoring of pathological wounds through the analysis of their glycolytic profile and the prediction of the outcome of surgical treatments.

## METHODS

*Animals.* C57Bl6/J female mice 8-14 weeks old (Charles River Laboratories) were used. 8-14 weeks old CX3CR1[+/gfp] female mice were purchased from The Jackson Laboratory. Mice were housed in the specific pathogen-free animal facility of the Humanitas Clinical and Research Center in individually ventilated cages. Procedures involving animals handling and care were conformed to protocols approved by the Humanitas Clinical and Research Center (Rozzano, Milan, Italy) in compliance with national (4D.L. N.116, G.U., suppl. 40, 18-2-1992; D.L. N. 26, G.U. 4-3-2014) and international law and policies (EEC Council Directive 2010/63/EU, OJ L 276/33, 22-09-2010; National Institutes of Health Guide for the Care and Use of Laboratory Animals, US National Research Council, 2011, ARRIVE guidelines). The study was approved by the Italian Ministry of Health (approval n. 71/2012-B, issued on the 09/03/2012). All efforts were made to minimize the number of animals used and their suffering.

*Excisional model of skin injury.* A full-thickness excisional skin injury model was performed as previously described[7]. Mice were anesthetized with 10μL/g of weight of Ketamine (final concentration 10mg/mL) and Xylazine (final concentration 1mg/mL) in saline buffer. As described[7], a full-thickness wound (from skin to underlying panniculus carnosus) was generated with disposable biopsy punch (8-mm diameter). Gentamicin was immediately applied to limit gross bacterial infections, not evident from histological examination carried out at different time points of the experiment. Mice were optionally treated with a solution of sodium dichloroacetate (DCA; Sigma-Aldrich Corp.), which was injected i.p. in 10-12 weeks-old C57BL/6J with a dosage of 300mg/Kg 1h before skin wounding. PBS or saline were used in control mice. As previously described[7], the skin wounds of each mouse were digitally photographed and measured during wound healing by tracing them on a transparency, calculated with the ImageJ-Fiji software, and the variations in time expressed as a percentage of the initial wound area.

*Incisional model of skin injury.* A full-thickness incisional skin injury model was performed with a scalpel in the centre region of the shaved back skin. We used C57Bl6/J mice of age between 8 and 14 weeks. Whole back skin was then hair-clipped and shaved with depilatory cream. Wound lesion was created by cutting the dorsal skin with a scalpel while pinching and pressing on a surface. The cut length was around 2-3mm, resulting in a final wound size of about 4-6mm. Mice older mice than 14 weeks showed more autofluorescence, which resulted in a longer time decay curve that could affect the exponential decay fitting in pH measurements and phasor analysis. DCA was used as described before. Rotenone (20μM, 50μl/mouse; Sigma-Aldrich Corp.) and Antimycin A (5μM, 50μl/mouse; Sigma-Aldrich Corp.) were finally resuspended in saline and i.d. injected around wound 1h before imaging analyses.

*Transplantable model of cancer.* Colon cancer cell line CT26 transfected with mCherry was prepared as described[48] and i.d. injected in a CD-1 nude mouse (1 × 10[6] cells in 100μL). After 3 days, 7-8×10[5] GFP[+] monocytes were isolated from the spleen of CX3CR1[+/gfp] mice and i.d. injected in the peritumoral area. Imaging analysis was conducted the day after their injection. The dermis was surgically exposed with a skin flap as previously reported[26] and mounted with tumour at the centre of the imaging chamber's window. A first acquisition in imaging modality at 740 nm was used to identify the two cell populations and create their corresponding threshold-based binary masks. The same *XYZ* area was then immediately acquired a second time in FLIM modality at 740 nm and analysed to obtain a global NADH/NAD$^+$ map of the tumour and peritumoral area. The two binary masks were then used extract NADH/NAD$^+$ maps of the corresponding cell populations. The NADH/NAD$^+$ maps were then linearized and the intensity profile was measured.

*Intravital imaging.* The implant procedure for the imaging chamber consists of pinching the dorsal skin on one block of the chamber after placing the mouse on one side, gently pressing the other block on the skin (Fig. S2). The pressure on the dorsal skin can be adjusted with the nuts, to avoid any restriction of blood flow. The imaging chamber is then mounted on a micrometre *XYZ* manipulator and lifted. The entire configuration creates a flat imaging window and greatly reduces motion artefacts due to the animal's breathing. Animal temperature was kept at 37°C with a heating pad (Fig. S2C), with mouse placed in lateral *decubitus.* Vital signs and anaesthesia level were regularly checked. Image mosaics were acquired with a 2-photon laser-scanning microscope (LaVision BioTec; Germany) in upright configuration for direct accessibility to the imaging window. Images were 200-250μm wide, with a pixel size of 0.8μm. The system is equipped with a Chameleon Ultra Ti:Sa laser (Coherent; USA), a 20X water-immersion objective (XLUMPLFLN-W, NA 1.0, Olympus; ; Germany), two GaAsP photomultipliers (H6780-20) and a TCSPC x16 FLIM detector (LaVision BioTec; Germany). Emission filters for the imaging modality were 525/50 and 609/54, with a dichroic mirror at 593nm.

*Measurement of glycolysis.* Images were acquired in FLIM modality with excitation wavelength 740nm and a power at sample range of 5 to 15mW, with a pixel dwell of 5 to 15μs. 3D Mosaics were acquired with a tile overlap of 0% to avoid artefacts in photon counts at edges and Z step size of 5 to 10μm. We applied a 3x3 binning to the images to increase the signal-to-noise ratio, losing spatial resolution but gaining analysis accuracy, and then calculated the phasor plot with TCSPC Phasor Analysis Plugin for Fiji/ImageJ. Phasor plot was calibrated with fluorescein (1mM; Sigma-Aldrich Corp.) solution (0.1M NaOH), NADH (100mM; Sigma-Aldrich Corp.) solution (0.1M MOPS buffer, pH 7) and second harmonic signal (SHG) from urea crystals (Bio-Rad Laboratories; USA) (n=5 measurements for both calibration points). We then designed an analysis tool that first divides the phasor cloud into five regions, corresponding to a gradient of prevailing anaerobic glycolysis in regions of lower lifetimes towards a region where more oxidative phosphorylation occurs[37]. To determine the number of divisions, we assumed that if there were a gradient, it would between a region of high anaerobic glycolysis and a flat region corresponding to the homeostatic condition, with an inflection point in the central region. To describe data with an inflection point at least four points are needed, but since the inflection point was likely to occur at the centre we took an odd number of bands, hence five was the minimum required. These bands were constructed from the major axis of the NADH phasor cloud[49]. Pixels belonging to each region are then associated to five discrete values of decreasing intensity, equally spaced from 0 to 100 (bins 0-20, 20-40, 40-60, 60-80, 80-100), starting with the highest values from anaerobic glycolysis. Quantification of pixel intensity is thus proportional to the relative increase in anaerobic glycolysis. Spatial distribution of this intensity from wound margin was then analysed with the mapping analysis described below. Values are expressed as difference from basal level of each mouse, as measured in a region distant from lesion margin.

*Measurement of hypoxia.* Hypoxyprobe Pimonidazole-HCl (Pimo) (Hypoxyprobe; USA) was dissolved to a concentration of 100mg/ml in PBS$^{++}$, and 40μL of Pimo were i.d. injected at perilesional area of the wound site. After 45 minutes, we injected 30μL of antipimonidazole monoclonal antibody conjugated to Dylight 549 (Hypoxyprobe; USA) at 60μg/ml. Images were acquired 45 minutes after the Ab injections in imaging modality, with an excitation wavelength of 780nm and a power at sample of 3-5mW. A line average of 2 was applied, for a total pixel dwell of 8μs. 3D Mosaics were acquired with a tile overlap of 0% to avoid artefacts in photon counts at edges and Z step size of 5 to 10μm. Images were then reconstructed with Fiji/ImageJ Stitching plugin and we measured the spatial distribution of fluorescence intensity with the mapping analysis described below.

*Measurement of pH.* 2',7'-Bis-(2-Carboxyethyl)-5-(and-6)-Carboxyfluorescein (BCECF) (ThermoFisher Scientific; USA) was dissolved in PBS$^{++}$ to a final stock concentration of 1.6mM and then to a working solution of 80μM in Ethanol 100%. 50μL of the working solution were applied topically to portion of skin inside the imaging chamber 3 times, waiting for complete dye take-up between the applications. Images were acquired in FLIM modality with excitation wavelength 820nm and a power at sample range of 5 to 15mW. To reach at least $10^3$ photons per pixel, acquisition lines were summed to reach a pixel dwell range 30-45μs. 3D Mosaics were acquired with a tile overlap of 0% to avoid artefacts in photon counts at edges and Z step size of 5 to 10μm. To obtain single lifetime images, images were fitted with the FLIMfit software tool developed at Imperial College London, using a single exponential and 3x3 binning.

To compute pH from the fluorophore's lifetime, we start from the Henderson-Hasselbach equation[1]

$$pH = pKa + \log \frac{[BCECF^-]}{[HBCECF]}$$

Measured lifetime $\tau$ at a certain point can be expressed as weighted average of the lifetimes of protonated and unprotonated fluorophores, respectively $\tau_{high}$ and $\tau_{low}$

$$\tau = \frac{n_{high}\,\tau_{high} + n_{low}\tau_{low}}{n_{high} + n_{low}}$$

then

$$\frac{n_{high}}{n_{low}} = \frac{\tau - \tau_{low}}{\tau_{high} - \tau}$$

so that the Henderson-Hasselbach equation finally reads

$$pH = pKa + \log \frac{\tau - \tau_{low}}{\tau_{high} - \tau}$$

An analysis script employs this formula to compute corresponding pH from the lifetime images. Determination of the unprotonated and protonated lifetimes was extracted from a calibration curve that characterizes the lifetime distribution of the fluorophore at different pHs. As previously reported[1,7], we corrected the lifetime with a multiplication factor of 0.9 to consider a different refractive index in tissue than solution. Contribution to the lifetime from Second Harmonic Generation of abundant skin collagen or autofluorescence may change the shorter or longer part of the fluorescence time decay. For these reasons we chose an excitation wavelength that minimizes both contributions, deconvolved the Instrument Response Function (IRF) measured from SHG-emitting samples and excluded longer times from the time decay window. Single pH images were then merged in a mosaic with Fiji/ImageJ Stitching plugin. Spatial distribution of pH was then analysed with the mapping analysis described below.

*Mapping analysis.* The analysis of spatial distribution of glycolysis, pH and hypoxia were performed with a set of Fiji/ImageJ macros (Supplementary Data File 1). In the case of pH, the analysis first linearized the wound front and perilesional area, divided the linearized image in consecutive, equally spaced, ROI bins and measured the average values in each bin. For NADH/NAD⁺ maps and Pimo images, we first linearized the wound front and perilesional area and then measured the intensity profile.

*Statistical Analysis.* Data analyses and graph construction were performed using Prism 7 software (GraphPad; USA). The sample sizes for technical replicates are presented in the figure legends, and samples were randomly assigned to groups for experiments. Data are presented as means $\pm$ SEM unless otherwise stated. For analysis of the statistical significance by Student *t*-test, the data distribution in each experiment was checked for normality using the D'Agostino-Pearson test. P value for glycolytic index between tumour cells and GFP⁺ peritumoral cells was calculated using an independent-sample two-sided *t* test (Fig. 5d). Linear regression was calculated with a method of least squares, while deviation from slope test was used to assess the presence of a gradient while imposing the minimum set of assumptions on the profile function (Fig. 2d-e, 3d and 5d for the analysis of the fraction of anaerobic glycolysis in both tumour (x < 0) and peritumoral GFP⁺ cells (x > 0). Unless otherwise stated, in this study P < 0.05 was considered significant (*P < 0.05, **P < 0.01, ***P < 0.001, and ****P < 0.0001).

## REFERENCES

1. Hanson, K.M. et al. Two-photon fluorescence lifetime imaging of the skin stratum corneum pH gradient. Biophys J 83, 1682-1690 (2002).
2. Singer, A.J. & Clark, R.A. Cutaneous wound healing. New Engl J Med 341, 738-746 (1999).
3. Eming, S.A., Krieg, T. & Davidson, J.M. Inflammation in wound repair: molecular and cellular mechanisms. J Invest Dermatol 127, 514-525 (2007).
4. Serrano, C.V., Jr. et al. pH dependence of neutrophil-endothelial cell adhesion and adhesion molecule expression. Am J Physiol 271, C962-970 (1996).
5. Paradise, R.K., Lauffenburger, D.A. & Van Vliet, K.J. Acidic extracellular pH promotes activation of integrin alpha(v)beta(3). PloS one 6, e15746 (2011).
6. Liu, Y., Kalen, A., Risto, O. & Wahlstrom, O. Fibroblast proliferation due to exposure to a platelet concentrate in vitro is pH dependent. Wound Repair Regen 10, 336-340 (2002).
7. Doni, A. et al. An acidic microenvironment sets the humoral pattern recognition molecule PTX3 in a tissue repair mode. J Exp Med 212, 905-925 (2015).
8. Knighton, D.R. et al. Oxygen tension regulates the expression of angiogenesis factor by macrophages. Science 221, 1283-1285 (1983).
9. Colegio, O.R. et al. Functional polarization of tumour-associated macrophages by tumour-derived lactic acid. Nature 513, 559-563 (2014).
10. Biswas, S.K. & Mantovani, A. Orchestration of metabolism by macrophages. Cell Metab 15, 432-437 (2012).
11. Schneider, L.A., Korber, A., Grabbe, S. & Dissemond, J. Influence of pH on woundhealing: a new perspective for wound-therapy? Arch Dermatol Res 298, 413-420 (2007).
12. Sharpe, J.R. et al. Progression of wound pH during the course of healing in burns. J Burn Care Res 34, e201-208 (2013).
13. Power, G., Moore, Z. & O'Connor, T. Measurement of pH, exudate composition and temperature in wound healing: a systematic review. J Wound Care 26, 381-397 (2017).
14. Percival, S.L., McCarty, S., Hunt, J.A. & Woods, E.J. The effects of pH on wound healing, biofilms, and antimicrobial efficacy. Wound Repair Regen 22, 174-186 (2014).

15. Haverkampf, S. et al. NHE1 expression at wound margins increases timedependently during physiological healing. Exp Dermatol 26, 124-126 (2017).

16. Leveen, H.H. et al. Chemical acidification of wounds. An adjuvant to healing and the unfavorable action of alkalinity and ammonia. Ann Surg 178, 745-753 (1973).

17. Snyder, R.J. Treatment of nonhealing ulcers with allografts. Clin Dermatol 23, 388-395 (2005).

18. Shukla, V.K., Shukla, D., Tiwary, S.K., Agrawal, S. & Rastogi, A. Evaluation of pH measurement as a method of wound assessment. J Wound Care 16, 291-294 (2007).

19. Palsson-McDermott, E.M. et al. Pyruvate kinase M2 regulates Hif-1alpha activity and IL-1beta induction and is a critical determinant of the warburg effect in LPS-activated macrophages. Cell Metabol 21, 65-80 (2015).

20. Gatenby, R.A. & Gillies, R.J. Why do cancers have high aerobic glycolysis? Nat Rev Cancer 4, 891-899 (2004).

21. Robey, I.F. et al. Bicarbonate increases tumor pH and inhibits spontaneous metastases. Cancer Res 69, 2260-2268 (2009).

22. Ganapathy-Kanniappan, S. & Geschwind, J.F. Tumor glycolysis as a target for cancer therapy: progress and prospects. Mol Cancer 12, 152 (2013).

23. Bohn, T. et al. Tumor immunoevasion via acidosis-dependent induction of regulatory tumor-associated macrophages. Nat Immunol 19, 1319-1329 (2018).

24. Rofstad, E.K., Mathiesen, B., Kindem, K. & Galappathi, K. Acidic extracellular pH promotes experimental metastasis of human melanoma cells in athymic nude mice. Cancer Res 66, 6699-6707 (2006).

25. Kato, Y., Nakayama, Y., Umeda, M. & Miyazaki, K. Induction of 103-kDa gelatinase/type IV collagenase by acidic culture conditions in mouse metastatic melanoma cell lines. JBiol Chem 267, 11424-11430 (1992).

26. Estrella, V. et al. Acidity generated by the tumor microenvironment drives local invasion. Cancer Res 73, 1524-1535 (2013).

27. Cardone, R.A., Casavola, V. & Reshkin, S.J. The role of disturbed pH dynamics and the Na+/H+ exchanger in metastasis. Nat Rev Cancer 5, 786-795 (2005).

28. Neri, D. & Supuran, C.T. Interfering with pH regulation in tumours as a therapeutic strategy. Nat Rev Drug Discov 10, 767-777 (2011).

29. Mantovani, A., Biswas, S.K., Galdiero, M.R., Sica, A. & Locati, M. Macrophage plasticity and polarization in tissue repair and remodelling. J Pathol 229, 176-185 (2013).

30. Mantovani, A., Sozzani, S., Locati, M., Allavena, P. & Sica, A. Macrophage polarization: tumor-associated macrophages as a paradigm for polarized M2 mononuclear phagocytes. Trends Immunol 23, 549-555 (2002).

31. Rinnenthal, J.L. et al. Parallelized TCSPC for dynamic intravital fluorescence lifetime imaging: quantifying neuronal dysfunction in neuroinflammation. PloS one 8, e60100 (2013).

32. Stringari, C. et al. Metabolic trajectory of cellular differentiation in small intestine by Phasor Fluorescence Lifetime Microscopy of NADH. Sci Rep 2, 568 (2012).

33. Bird, D.K. et al. Metabolic mapping of MCF10A human breast cells via multiphoton fluorescence lifetime imaging of the coenzyme NADH. Cancer Res 65, 8766-8773 (2005).

34. Yu, Q. & Heikal, A.A. Two-photon autofluorescence dynamics imaging reveals sensitivity of intracellular NADH concentration and conformation to cell physiology at the single-cell level. JPhotochem Photobiol B 95, 46-57 (2009).

35. Gomez, C.A. et al. Phasor analysis of NADH FLIM identifies pharmacological disruptions to mitochondrial metabolic processes in the rodent cerebral cortex. PloS one 13, e0194578 (2018).

36. Digman, M.A., Caiolfa, V.R., Zamai, M. & Gratton, E. The phasor approach to fluorescence lifetime imaging analysis. Biophys J 94, L14-16 (2008).

37. Stringari, C., Nourse, J.L., Flanagan, L.A. & Gratton, E. Phasor fluorescence lifetime microscopy of free and protein-bound NADH reveals neural stem cell differentiation potential. PloS one 7, e48014 (2012).

38. Blacker, T.S. et al. Separating NADH and NADPH fluorescence in live cells and tissues using FLIM. Nat Commun 5, 3936 (2014).

39. Kalinina, S. et al. Correlative NAD(P)H-FLIM and oxygen sensing-PLIM for metabolic mapping. J Biophotonics 9, 800-811 (2016).

40. Pokusa, M. & Kralova Trancikova, A. FLIM analysis of intracellular markers associated with the development of Parkinson's disease in cellular model. Physiol Res 67, S673-S683 (2018).

41. de Graaf, A.O. et al. Bcl-2 protects against apoptosis induced by antimycin A and bongkrekic acid without restoring cellular ATP levels. Biochim Biophys Acta 1554, 57-65 (2002).

42. Subramani, K. et al. Mitochondrial targeting by dichloroacetate improves outcome following hemorrhagic shock. Sci Rep 7, 2671 (2017).

43. Anderson, M., Moshnikova, A., Engelman, D.M., Reshetnyak, Y.K. & Andreev, O.A. Probe for the measurement of cell surface pH in vivo and ex vivo. Proc Natl Acad Sci USA 113, 8177-8181 (2016).

44. Lin, Y., Wu, T.Y. & Gmitro, A.F. Error analysis of ratiometric imaging of extracellular pH in a window chamber model. J Biomed Opt 17, 046004 (2012).

45. Bao, B. et al. In vivo imaging and quantification of carbonic anhydrase IX expression as an endogenous biomarker of tumor hypoxia. PloS one 7, e50860 (2012).

46. Cheng, Z. et al. Near-infrared fluorescent deoxyglucose analogue for tumor optical imaging in cell culture and living mice. Bioconjug Chem 17, 662-669 (2006).

47. Jones, J.D., Ramser, H.E., Woessner, A.E. & Quinn, K.P. In vivo multiphoton microscopy detects longitudinal metabolic changes associated with delayed skin wound healing. Commun Biol 1, 198 (2018).

48. Erreni, M. et al. The Fractalkine-Receptor Axis Improves Human Colorectal Cancer Prognosis by Limiting Tumor Metastatic Dissemination. J Immunol 196, 902-914 (2016).

49. Ranjit, S., Malacrida, L., Jameson, D.M. & Gratton, E. Fit-free analysis of fluorescence lifetime imaging data using the phasor approach. Nat Protoc 13, 1979-2004 (2018).

## Claims

1. A method for the quantitative three-dimensional (3D) imaging of the metabolic status of a lesion, such method comprising:

   a) acquiring fluorescence lifetime imaging microscopy (FLIM) data of an anaerobic glycolysis marker of the lesion;
   b) subjecting the data relative to the anaerobic glycolysis marker acquired in step a) to a phasor analysis, thereby obtaining a phasor diagram;
   c) using the phasor diagram obtained in step b) to computationally generate a quantitative 3D image of the anaerobic glycolysis status of the lesion,

   wherein the anaerobic glycolysis marker is selected from the list of the NADH/NAD+ ratio, NAD(P)H/NAD+ ratio, the NADH/FAD ratio, the NAD(P)H/FAD ratio and the FADH/FAD ratio and wherein step c) when applied to the data relative to anaerobic glycolysis marker consists in dividing the data in a number of regions corresponding to different intervals of NADH, NAD(P)H or FADH lifetimes and assigning each pixel belonging to the same region a single intensity value correlated to such lifetime interval.

2. The method of claim 1, further comprising:

   d) acquiring, simultaneously to the data acquired in step a), FLIM data relative to a second metabolic marker of the lesion;
   e) computationally generate a quantitative 3D image with the data acquired in step d);
   f) overlay the images obtained in steps c) and e) thereby obtaining a 3D image of the metabolic status of a lesion.

3. The method of claim 2, wherein the second metabolic marker is selected from the list of pH, oxygen tension, glucose, pyruvate and lactate.

4. The method of claims 1 to 3, wherein the number of regions is chosen from the list of at least 3, at least 5 and 5.

5. The method of claims 2-4, wherein in step e) the data relative to pH is processed by fitting the fluorescence lifetime of each pixel to a pH value by reference to a standard calibration scale.

6. The method of claims 1-5 wherein the FLIM is selected from the list of single photon FLIM, 2-photon FLIM and multiphoton FLIM.

7. The method of claims 1 to 6 wherein the lesion is selected from the list of a wound, an internal wound, a cutaneous wound, a pathologic wound, an ulcer, a scar, a solid tumour and a tumour-draining lymph node.

8. The method of claim 7, wherein the solid tumour is selected from the list of skin tumour, melanoma, colon tumour, soft tissue sarcoma, visceral sarcoma, fibrosarcoma, liver tumour and stomach tumour.

## Patentansprüche

1. Verfahren zur quantitativen dreidimensionalen (3D-) Darstellung des Stoffwechselstatus einer Läsion, wobei das Verfahren umfasst:

   a) Erfassen von Fluoreszenzlebensdauer-Bildgebungsmikroskopie-(ELIM)-Daten eines Markers für die anaerobe Glykolyse der Läsion;
   b) Unterziehen der in Schritt a) erfassten Daten bezüglich des Markers für die anaerobe Glykolyse einer Phasoranalyse, wodurch ein Phasordiagramm erhalten wird;
   c) Verwenden des in Schritt b) erhaltenen Phasordiagramms zur rechnerischen Erzeugung eines quantitativen 3D-Bilds des Status der anaeroben Glykolyse der Läsion,

   wobei der Marker für die anaerobe Glykolyse aus der Liste aus dem NADH/NAD+-Verhältnis, dem NAD(P)H/NAD+-Verhältnis, dem NADH/FAD-Verhältnis, dem NAD(P)H/FAD-Verhältnis und dem FADH/FAD-Verhältnis ausgewählt wird und wobei Schritt c), wenn er auf die Daten bezüglich des Markers für die anaerobe Glykolyse angewendet wird, darin besteht, die Daten in eine Anzahl von Bereichen zu unterteilen, die verschiedenen Zeitspannen der NADH-, NAD(P)H- oder FADH-Lebensdauer entsprechen, und jedes Pixel, das zu demselben Bereich gehört, einem einzelnen Intensitätswert zuzuweisen, der mit einer solchen Lebensdauer-Zeitspanne korreliert ist.

2. Verfahren nach Anspruch 1, ferner umfassend:

   d) Erfassen, gleichzeitig mit den in Schritt a) erfassten Daten, von FLIM-Daten bezüglich eines zweiten Stoffwechselmarkers der Läsion;
   e) rechnerisches Erzeugen eines quantitativen 3D-Bilds mit den in Schritt d) erfassten Daten;
   f) Überlagern der in Schritt c) und e) erhaltenen Daten, wodurch ein 3D-Bild des Stoffwechselstatus einer Läsion erhalten wird.

3. Verfahren nach Anspruch 2, wobei der zweite Stoffwechselmarker aus der Liste aus pH, Sauerstoffspannung, Glukose, Pyruvat und Laktat ausgewählt wird.

4. Verfahren nach Anspruch 1 bis 3, wobei die Anzahl der Bereiche aus der Liste aus wenigstens 3, wenigstens 5 und 5 ausgewählt wird.

5. Verfahren nach Anspruch 2 bis 4, wobei in Schritt e) die Daten bezüglich des pH verarbeitet werden, indem die Fluoreszenzlebensdauer jedes Pixels durch Bezugnahme auf eine Standardkalibrierungsskala an einen pH-Wert angepasst wird.

6. Verfahren nach Anspruch 1 bis 5, wobei die FLIM aus der Liste aus Ein-Photonen-FLIM, 2-Photonen-FLIM und Multi-Photonen-FLIM ausgewählt wird.

7. Verfahren nach Anspruch 1 bis 6, wobei die Läsion aus der Liste aus einer Wunde, einer inneren Wunde, einer Hautwunde, einer pathologischen Wunde, einem Geschwür, einer Narbe, einem soliden Tumor und einem tumordrainierenden Lymphknoten ausgewählt wird.

8. Verfahren nach Anspruch 7, wobei der solide Tumor aus der Liste aus Hauttumor, Melanom, Dickdarmtumor, Weichteilsarkom, viszeralem Sarkom, Fibrosarkom, Lebertumor und Magentumor ausgewählt wird.

**Revendications**

1. Procédé d'imagerie tri-dimensionnelle (3D) quantitative du statut métabolique d'une lésion, ce procédé comprenant les étapes consistant à :

   a) acquérir les données de microscopie d'imagerie de durée de vie de fluorescence (FLIM) d'un marqueur de glycolyse anaérobie de la lésion ;
   b) soumettre les données relatives au marqueur de glycolyse anaérobie acquises dans l'étape a) à une analyse de phaseur, obtenant ainsi un diagramme de phaseur ;
   c) utiliser le diagramme de phaseur obtenu dans l'étape b) pour produire computationnellement une image 3D quantitative du statut de glycolyse anaérobie de la lésion,

   le marqueur de glycolyse anaérobie étant choisi de la liste du rapport NADH/NAD+, du rapport NAD(P)H/NAD+, du rapport NADH/FAD, du rapport NAD(P)H/FAD et du rapport FADH/FAD et l'étape c) quand elle est appliquée aux données relatives au marqueur de glycolyse anaérobie consistant en la division des données dans un nombre de régions correspondant à des intervalles différents de durées de vie de NADH, NAD(P)H ou FADH et en assignant chaque pixel appartenant à la même région à une seule valeur d'intensité corrélée à cet intervalle de durée de vie.

2. Procédé selon la revendication 1, comprenant en outre :

   d) d'acquérir, simultanément aux données acquises dans l'étape a), les données FLIM relatives à un second marqueur métabolique de la lésion ;
   e) de produire computationnellement une image 3D quantitative avec les données acquises dans l'étape d) ;
   f) de superposer les images obtenues dans les étapes c) et e) obtenant ainsi une image 3D du statut métabolique de la lésion.

3. Procédé selon la revendication 2, dans lequel le second marqueur métabolique est choisi dans la liste du pH, de la tension d'oxygène, du glucose, du pyruvate et du lactate.

4. Procédé selon les revendications 1 à 3, dans lequel le nombre de régions est choisi dans la liste d'au moins 3, d'au moins 5 et de 5.

5. Procédé selon les revendications 2 à 4, dans lequel dans l'étape e) les données relatives au pH sont traitées par ajustement de la durée de vie de fluorescence de chaque pixel à une valeur de pH par référence à une échelle de calibration standard.

6. Procédé selon les revendications 1 à 5, dans lequel le FLIM est choisi dans la liste du FLIM à un seul photon, du FLIM à 2 photons et du FLIM multi-photons.

7.  Procédé selon les revendications 1 à 6, dans lequel la lésion est choisie dans la liste d'une blessure, d'une blessure interne, d'une blessure cutanée, d'une blessure pathologique, d'un ulcère, d'une cicatrice, d'une tumeur solide et d'un nœud lymphatique drainant une tumeur.

8.  Procédé selon la revendication 7, dans lequel la tumeur solide est choisie dans la liste d'une tumeur de la peau, d'un mélanome, d'une tumeur du côlon, d'un sarcome de tissu mou, d'un sarcome viscéral, d'un fibrosarcome, d'une tumeur du foie et d'une tumeur de l'estomac.

**Figure 1**

**Figure 2**

**Figure 3**

**Figure 4**

Figure 5

**Figure 6**

**Two-phase exponential decay**

**untreated**
Percentage of protein-bound NADH: 93.67 ± 0.74 %
Lifetimes: $\tau_1$ = 2.69 ± 0.89 ns  $\tau_2$ = 0.48 ± 0.02 ns

**DCA**
Percentage of protein-bound NADH: 90.92 ± 3.03 %
Lifetimes: $\tau_1$ = 2.05 ± 0.75 ns  $\tau_2$ = 0.54 ± 0.04 ns

**Figure 7**

**Figure 8**

**EP 4 132 347 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012276578 A1

**Non-patent literature cited in the description**

- **HANSON, K.M. et al.** Two-photon fluorescence lifetime imaging of the skin stratum corneum pH gradient. *Biophys J,* 2002, vol. 83, 1682-1690
- **SINGER, A.J. ; CLARK, R.A.** Cutaneous wound healing. *New Engl J Med,* 1999, vol. 341, 738-746
- **EMING, S.A. ; KRIEG, T. ; DAVIDSON, J.M.** Inflammation in wound repair: molecular and cellular mechanisms. *J Invest Dermatol,* 2007, vol. 127, 514-525
- **SERRANO, C.V., JR. et al.** pH dependence of neutrophil-endothelial cell adhesion and adhesion molecule expression. *Am J Physiol,* 1996, vol. 271, C962-970
- **PARADISE, R.K. ; LAUFFENBURGER, D.A. ; VAN VLIET, K.J.** Acidic extracellular pH promotes activation of integrin alpha(v)beta(3). *PloS one,* 2011, vol. 6, e15746
- **LIU, Y. ; KALEN, A. ; RISTO, O. ; WAHLSTROM, O.** Fibroblast proliferation due to exposure to a platelet concentrate in vitro is pH dependent. *Wound Repair Regen,* 2002, vol. 10, 336-340
- **DONI, A. et al.** An acidic microenvironment sets the humoral pattern recognition molecule PTX3 in a tissue repair mode. *J Exp Med,* 2015, vol. 212, 905-925
- **KNIGHTON, D.R. et al.** Oxygen tension regulates the expression of angiogenesis factor by macrophages. *Science,* 1983, vol. 221, 1283-1285
- **COLEGIO, O.R. et al.** Functional polarization of tumour-associated macrophages by tumour-derived lactic acid. *Nature,* 2014, vol. 513, 559-563
- **BISWAS, S.K. ; MANTOVANI, A.** Orchestration of metabolism by macrophages. *Cell Metab,* 2012, vol. 15, 432-437
- **SCHNEIDER, L.A. ; KORBER, A. ; GRABBE, S. ; DISSEMOND, J.** Influence of pH on woundhealing: a new perspective for wound-therapy?. *Arch Dermatol Res,* 2007, vol. 298, 413-420
- **SHARPE, J.R. et al.** Progression of wound pH during the course of healing in burns. *J Burn Care Res,* 2013, vol. 34, e201-208

- **POWER, G. ; MOORE, Z. ; O'CONNOR, T.** Measurement of pH, exudate composition and temperature in wound healing: a systematic review. *J Wound Care,* 2017, vol. 26, 381-397
- **PERCIVAL, S.L. ; MCCARTY, S. ; HUNT, J.A. ; WOODS, E.J.** The effects of pH on wound healing, biofilms, and antimicrobial efficacy. *Wound Repair Regen,* 2014, vol. 22, 174-186
- **HAVERKAMPF, S. et al.** NHE1 expression at wound margins increases timedependently during physiological healing. *Exp Dermatol,* 2017, vol. 26, 124-126
- **LEVEEN, H.H. et al.** Chemical acidification of wounds. An adjuvant to healing and the unfavorable action of alkalinity and ammonia. *Ann Surg,* 1973, vol. 178, 745-753
- **SNYDER, R.J.** Treatment of nonhealing ulcers with allografts. *Clin Dermatol,* 2005, vol. 23, 388-395
- **SHUKLA, V.K. ; SHUKLA, D. ; TIWARY, S.K. ; AGRAWAL, S. ; RASTOGI, A.** Evaluation of pH measurement as a method of wound assessment. *J Wound Care,* 2007, vol. 16, 291-294
- **PALSSON-MCDERMOTT, E.M. et al.** Pyruvate kinase M2 regulates Hif-1alpha activity and IL-1beta induction and is a critical determinant of the warburg effect in LPS-activated macrophages. *Cell Metabol,* 2015, vol. 21, 65-80
- **GATENBY, R.A. ; GILLIES, R.J.** Why do cancers have high aerobic glycolysis?. *Nat Rev Cancer,* 2004, vol. 4, 891-899
- **ROBEY, I.F. et al.** Bicarbonate increases tumor pH and inhibits spontaneous metastases. *Cancer Res,* 2009, vol. 69, 2260-2268
- **GANAPATHY-KANNIAPPAN, S. ; GESCHWIND, J.F.** Tumor glycolysis as a target for cancer therapy: progress and prospects. *Mol Cancer,* 2013, vol. 12, 152
- **BOHN, T. et al.** Tumor immunoevasion via acidosis-dependent induction of regulatory tumor-associated macrophages. *Nat Immunol,* 2018, vol. 19, 1319-1329

- **ROFSTAD, E.K. ; MATHIESEN, B. ; KINDEM, K. ; GALAPPATHI, K.** Acidic extracellular pH promotes experimental metastasis of human melanoma cells in athymic nude mice. *Cancer Res,* 2006, vol. 66, 6699-6707
- **KATO, Y. ; NAKAYAMA, Y. ; UMEDA, M. ; MIYAZAKI, K.** Induction of 103-kDa gelatinase/type IV collagenase by acidic culture conditions in mouse metastatic melanoma cell lines. *JBiol Chem,* 1992, vol. 267, 11424-11430
- **ESTRELLA, V. et al.** Acidity generated by the tumor microenvironment drives local invasion. *Cancer Res,* 2013, vol. 73, 1524-1535
- **CARDONE, R.A. ; CASAVOLA, V ; RESHKIN, S.J.** The role of disturbed pH dynamics and the Na+/H+ exchanger in metastasis. *Nat Rev Cancer,* 2005, vol. 5, 786-795
- **NERI, D. ; SUPURAN, C.T.** Interfering with pH regulation in tumours as a therapeutic strategy. *Nat Rev Drug Discov,* 2011, vol. 10, 767-777
- **MANTOVANI, A. ; BISWAS, S.K. ; GALDIERO, M.R. ; SICA, A. ; LOCATI, M.** Macrophage plasticity and polarization in tissue repair and remodelling. *J Pathol,* 2013, vol. 229, 176-185
- **MANTOVANI, A. ; SOZZANI, S. ; LOCATI, M. ; AL-LAVENA, P. ; SICA, A.** Macrophage polarization: tumor-associated macrophages as a paradigm for polarized M2 mononuclear phagocytes. *Trends Immunol,* 2002, vol. 23, 549-555
- **RINNENTHAL, J.L. et al.** Parallelized TCSPC for dynamic intravital fluorescence lifetime imaging: quantifying neuronal dysfunction in neuroinflammation. *PloS one,* 2013, vol. 8, e60100
- **STRINGARI, C. et al.** Metabolic trajectory of cellular differentiation in small intestine by Phasor Fluorescence Lifetime Microscopy of NADH. *Sci Rep,* 2012, vol. 2, 568
- **BIRD, D.K. et al.** Metabolic mapping of MCF10A human breast cells via multiphoton fluorescence lifetime imaging of the coenzyme NADH. *Cancer Res,* 2005, vol. 65, 8766-8773
- **YU, Q. ; HEIKAL, A.A.** Two-photon autofluorescence dynamics imaging reveals sensitivity of intracellular NADH concentration and conformation to cell physiology at the single-cell level. *JPhotochem Photobiol B,* 2009, vol. 95, 46-57
- **GOMEZ, C.A. et al.** Phasor analysis of NADH FLIM identifies pharmacological disruptions to mitochondrial metabolic processes in the rodent cerebral cortex. *PloS one,* 2018, vol. 13, e0194578
- **DIGMAN, M.A. ; CAIOLFA, V.R. ; ZAMAI, M. ; GRATTON, E.** The phasor approach to fluorescence lifetime imaging analysis. *Biophys J,* 2008, vol. 94, L14-16
- **STRINGARI, C. ; NOURSE, J.L. ; FLANAGAN, L.A. ; GRATTON, E.** Phasor fluorescence lifetime microscopy of free and protein-bound NADH reveals neural stem cell differentiation potential. *PloS one,* 2012, vol. 7, e48014
- **BLACKER, T.S. et al.** Separating NADH and NADPH fluorescence in live cells and tissues using FLIM. *Nat Commun,* 2014, vol. 5, 3936
- **KALININA, S. et al.** Correlative NAD(P)H-FLIM and oxygen sensing-PLIM for metabolic mapping. *J Biophotonics,* 2016, vol. 9, 800-811
- **POKUSA, M. ; KRALOVA TRANCIKOVA, A.** FLIM analysis of intracellular markers associated with the development of Parkinson's disease in cellular model. *Physiol Res,* 2018, vol. 67, S673-S683
- **DE GRAAF, A.O. et al.** Bcl-2 protects against apoptosis induced by antimycin A and bongkrekic acid without restoring cellular ATP levels. *Biochim Biophys Acta,* 2002, vol. 1554, 57-65
- **SUBRAMANI, K. et al.** Mitochondrial targeting by dichloroacetate improves outcome following hemorrhagic shock. *Sci Rep,* 2017, vol. 7, 2671
- **ANDERSON, M. ; MOSHNIKOVA, A. ; ENGELMAN, D.M. ; RESHETNYAK, Y.K. ; ANDREEV, O.A.** Probe for the measurement of cell surface pH in vivo and ex vivo. *Proc Natl Acad Sci USA,* 2016, vol. 113, 8177-8181
- **LIN, Y. ; WU, T.Y. ; GMITRO, A.F.** Error analysis of ratiometric imaging of extracellular pH in a window chamber model. *J Biomed Opt,* 2012, vol. 17, 046004
- **BAO, B. et al.** In vivo imaging and quantification of carbonic anhydrase IX expression as an endogenous biomarker of tumor hypoxia. *PloS one,* 2012, vol. 7, e50860
- **CHENG, Z. et al.** Near-infrared fluorescent deoxyglucose analogue for tumor optical imaging in cell culture and living mice. *Bioconjug Chem,* 2006, vol. 17, 662-669
- **JONES, J.D. ; RAMSER, H.E. ; WOESSNER, A.E. ; QUINN, K.P.** In vivo multiphoton microscopy detects longitudinal metabolic changes associated with delayed skin wound healing. *Commun Biol,* 2018, vol. 1, 198
- **ERRENI, M. et al.** The Fractalkine-Receptor Axis Improves Human Colorectal Cancer Prognosis by Limiting Tumor Metastatic Dissemination. *J Immunol,* 2016, vol. 196, 902-914
- **RANJIT, S. ; MALACRIDA, L. ; JAMESON, D.M. ; GRATTON, E.** Fit-free analysis of fluorescence lifetime imaging data using the phasor approach. *Nat Protoc,* 2018, vol. 13, 1979-2004